# EUROPÄISCHE PATENTANMELDUNG

(11) **EP 1 795 939 A1**
(43) Veröffentlichungstag der Anmeldung: **13.06.2007**
(21) Anmeldenummer: 05026775.6
(22) Anmeldetag: 08.12.2005
(51) Int. Cl.: G02B 21/22

(54) **Verfahren zum Betreiben eines optischen Systems und optisches System**

(71) Anmelder: Swiss Medical Technology GmbH, 9443 Widnau (CH)
(72) Erfinder: Die Erfindernennung liegt noch nicht vor
(74) Vertreter: Zwicker, Jörk

(57) **Zusammenfassung**

Die Erfindung betrifft ein Verfahren zum Betreiben eines optischen Systems (1) sowie ein optisches System. Be dem Verfahren wird mittels eines von dem optischen System (1) umfaßten Mikroskops (2) ein Untersuchungsobjekt in eine Bildebene räumlich abgebildet, wobei das Mikroskop (2) mindestens ein Linsensystem, welches einem jeweiligen Strahlengang (3,4) zugeordnet ist, und eine dem Strahlengang oder Strahlengängen (3,4) zugeordnete Sammellinsenanordnung (11) aufweist, wobei das mindestens eine Linsensystem eine erste Linsenanordnung (3a;4a) und eine zwischen der ersten Linsenanordnung (3a;4a) und des Sammellinsenanordnung (11) angeordnete, zweite Linsenanordnung (3b;4b) umfaßt. Weiterhin umfaßt das Verfahren die folgenden Schritte: Erfaßen einer Benutzereingabe zum Einstellen einer Fokuseinstellung und / oder eines Zoom-Faktors des Mikroskops (2) mit Hilfe einer Eingabeeinrichtung (21); Erzeugen von der erfaßten Benutzereingabe zugeordneten Steuersignalen zum Einstellen der Fokuseinstellung und / oder des Zoom-Faktors in einer an die Eingabeeinrichtung (21) gekoppelten Steuereinrichtung (20), wobei die Steuersignale Vorabeinstellungsdaten entsprechend gebildet werden, die in einer mit der Steuereinrichtung (20) verbundenen Speichereinrichtung (22) vorab gespeichert sind; Übertragen der Steuersignale an eine oder mehrere jeweiliger motorgetriebener Verstelleinrichtungen (7-10,13) zum Verlagern der ersten Linsenanordnung (3a;4a), der zweiten Linsenanordnung (3b;4b) und / oder der Sammellinsenanordnung (11); und Einstellen der Fokuseinstellung und / oder des Zoom-Faktors des Mikroskops (2) entsprechend den Steuersignalen, indem die erste Linsenanordnung (3a;4a), die zweite Linsenanordnung (3b;4b) und / oder die Sammellinsenanordnung (11) mit Hilfe der jeweiligen motorgetriebenen Verstelleinrichtung (7-10,13) verlagert werden.

## Beschreibung

Die Erfindung betrifft ein Verfahren zum Betreiben eines optischen Systems und ein optisches System.

### Hintergrund der Erfindung

Optische Systeme, beispielsweise Stereomikroskope, werden in verschiedensten Anwendungen dazu genutzt, eine räumliche Abbildung eines Untersuchungsobjektes zu erzeugen. Beispielsweise werden solche optischen Systeme in medizinischen Operationsmikroskopen eingesetzt, um einen Operationsbereich einzusehen.

Die räumliche Abbildung des Untersuchungsobjektes wird unter Verwendung des Stereomikroskops erzeugt, bei dem es sich um einen Vertreter aus der Geräteklasse von Auflichtmikroskopen handelt. Das Untersuchungsobjekt wird von außen beleuchtet. Es sind üblicherweise zwei getrennte Strahlengänge gebildet, die ihrerseits mit einem jeweils zugeordneten Linsensystem ausgestattet sind. Die optischen Achsen der beiden Strahlengänge verlaufen durch eine den Linsensystemen in Richtung des üntersuchungsobjektes vorgelagerte Sammellinsenanordnung. Sowohl die Linsensysteme als auch die Sammellinsenanordnung können mehrere optische Linsen umfassen. Die verschiedenen optischen Linsen werden genutzt, um einen Fokus sowie einen Zoom-Faktor des Stereomikroskops einzustellen. Zu diesem Zweck werden die Linsen einzeln oder in Gruppen entlang der jeweils zugeordneten optischen Achse verschoben.

Die Verschiebung optischer Elemente zur Fokuseinstellung oder zur Einstellung des Zoom-Faktors kann mit Hilfe motorgetriebener Verstelleinrichtungen ausgeführt werden, die das optische Element in eine gewünschte Position entlang der optischen Achse verlagern. Im Gegensatz zu der auch bekannten Verlagerung optischer Elemente per Hand werden solche motorgetriebenen Verstelleinrichtungen mit einem Betätigungsmittel, beispielsweise einer die Spannungs- oder Stromversorgung regelnden Steuerung, vom Benutzer betätigt. Solange der Benutzer das Betätigungsmittel einschaltet, verschiebt sich die Linse entlang der optischen Achse. Diese Bewegung stoppt der Benutzer, indem das Betätigungsmittel ausgeschaltet wird.

In dem Dokument DE 103 23 629 A1 ist ein Wandelfeld-Linearmotor beschrieben, mit dem in einer Hülse angeordnete optische Elemente, beispielsweise eine optische Linse, entlang der optischen Achse verschoben werden können. Dieses wird erreicht mittels eines entlang der optischen Achse bewegten magnetischen Wandelfeldes. Das magnetische Wandelfeld und somit auch die Verschiebestellungen der optischen Elemente sind sehr fein einstellbar. Der bekannte Wandelfeld-Linearmotor unterstützt die Miniaturisierung von optischen Systemen.

### Zusammenfassung der Erfindung

Aufgabe der Erfindung ist es, ein Verfahren zum Betreiben eines optischen Systems mit einem Mikroskop sowie ein optisches System zu schaffen, bei denen die Bedienfreundlichkeit verbessert ist, insbesondere hinsichtlich einer einfachen Bedienung und einer gezielten Positionierung der optischen Elemente innerhalb eines optischen Kanals.

Diese Aufgabe wird erfindungsgemäß durch ein Verfahren zum Betreiben eines optischen Systems mit einem Mikroskop nach dem unabhängigen Anspruch 1 sowie ein optisches System mit einem Mikroskop nach dem unabhängigen Anspruch 10 gelöst.

Die Erfindung umfaßt den Gedanken, eines optischen Systems so zu betreiben, daß als Reaktion auf eine Benutzereingabe zum Verändern einer Fokuseinstellung und / oder eines Zoom-Faktors des Mikroskops eine gewünschte Fokuseinstellung und / oder ein gewünschter Zoom-Faktor mit Hilfe von motorgetriebenen Verstelleinrichtungen automatisch erzeugt werden, indem als Reaktion auf die erfaßte Benutzereingabe in einer Steuereinrichtung Steuersignale entsprechend gespeicherten Vorabeinstellungen erzeugt und mit Hilfe der jeweiligen motorgetriebenen Verstelleinrichtungen in Bewegungen der optischen Elemente des Mikroskops umgesetzt werden. Zu diesem Zweck umfassen die gespeicherten Vorabeinstellungen elektronische Informationen über Positionen von optischen Elementen, insbesondere Linsen, des Mikroskops für bestimmte Fokuseinstellungen und / oder Zoom-Faktoren. Auf diese Weise kann in Abhängigkeit von der erfaßten Benutzereingabe das Mikroskop unter Rückgriff auf die gespeicherten Vorabeinstellungen automatisch so eingestellt werden, daß eine bestimmte Fokuseinstellung und / oder ein bestimmter Zoom-Faktor gegeben sind. Die Vorabeinstellungen sind für das optische System vorab ermittelt worden, beispielsweise mit Hilfe von Messungen auf einer optischen Bank, und in Form elektronischer Daten gespeichert.

Der Benutzer muß nicht die optischen Elemente selbständig entlang der optischen Achse verschieben und eine optimale Positionierung suchen, wie dies im Stand der Technik vorgesehen ist. Vielmehr gibt der Benutzer lediglich eine gewünschte Fokuseinstellung und / oder einen gewünschten Zoom-Faktor vor, woraufhin das optische System selbsttätig unter Berücksichtigung der gespeicherten Vorabeinstellungen Fokus- / Zoom-Faktoreinstellung vornimmt. Es ist auch keine Verschiebung der Linsen von Hand notwendig.

Eine zweckmäßige Ausgestaltung der Erfindung sieht vor, daß beim Einstellen der Fokuseinstellung eine grobe Fokuseinstellung ausgeführt wird, indem von den Steuersignalen umfaßte Grobsteuersignale in der Steuereinrichtung erzeugt und von der Steuereinrichtung an die motorgetriebene Verstelleinrichtung der Sammellinsenanordnung übertragen werden und die Sammellinsenanordnung mit Hilfe der motorgetriebenen Verstelleinrichtung der Sammellinsenanordnung den Grobsteuersignalen entsprechend verlagert wird. Auf unkomplizierte Weise ist so eine separate Grobeinstellung des Fokus ermöglicht, wahlweise als Vorstufe für eine anschließende Feinabstimmung des Fokus.

Bei einer bevorzugten Ausführungsform der Erfindung kann vorgesehen sein, daß beim Einstellen der Fokuseinstellung eine feine Fokuseinstellung ausgeführt wird, indem von den Steuersignalen umfaßte Feinsteuersignale in der Steuereinrichtung erzeugt und von der Steuereinrichtung an die jeweilige motorgetriebene Verstelleinrichtung der ersten Linsenanordnung in einem oder allen Strahlengängen übertragen werden und die erste Linsenanordnung in einem oder allen Strahlengängen mit Hilfe der jeweiligen motorgetriebenen Verstelleinrichtung den Feinsteuersignalen entsprechend verlagert wird. Hierdurch ist eine optimierte Fokuseinstellung mittels einer feinen Abstufung der Linsenpositionierung ermöglicht.

Bei einer vorteilhaften Ausgestaltung der Erfindung ist vorgesehen, daß das Einstellen des Zoom-Faktors ausgeführt wird, indem von den Steuersignalen umfaßte Zoomsteuersignale in der Steuereinrichtung erzeugt und von der Steuereinrichtung an die jeweilige motorgetriebene Verstelleinrichtung der zweiten Linsenanordnung in einem oder allen Strahlengängen übertragen werden und die zweite Linsenanordnung in einem oder allen Strahlengängen mit Hilfe der jeweiligen motorgetriebenen Verstelleinrichtung den Zoomsteuersignalen entsprechend verlagert wird. Hierdurch ist eine für den Benutzer auf einfache Weise vorgebbare Einstellung eines gewünschten Zoom-Faktors ermöglicht. Auch im Umgang mit komplexen optischen Systemen ungeübte Benutzer können so ohne weiteres einen vorgegebenen Zoom-Faktor einstellen.

Der Umfang der zu speichernden elektronischen Informationen für die Vorabeinstellungsdaten wird bei einer zweckmäßigen Ausführungsform der Erfindung dadurch minimiert, daß beim Erzeugen der Steuersignale zum Einstellen der Fokuseinstellung und / oder des Zoom-Faktors in der Steuereinrichtung eine Interpolation zwischen ausgewählten Vorabeinstellungsdaten der gespeicherten Vorabeinstellungsdaten ausgeführt wird. Mittels Interpolation können auch Fokuseinstellungen und / oder Einstellungen von Zoom-Faktoren ausgeführt werden, für die die zugehörige Positionierung der Linsen nicht vorab gemessen wurde. Üblicherweise wird eine lineare Interpolation zwischen den herangezogenen Vorabeinstellungsdaten durchgeführt.

Bei einer zweckmäßigen Weiterbildung der Erfindung kann vorgesehen sein, daß die Sammellinsenanordnung, die erste Linsenanordnung und / oder die zweite Linsenanordnung beim Verlagern entsprechend der Steuersignale mit Hilfe der jeweiligen motorgetriebenen Verstelleinrichtung linear verschoben werden. Zur Ausführung des Verlagerns wird bevorzugt ein Linearmotor mit den Steuersignalen beaufschlagt. Eine derartige Bewegungsgestaltung beim Verlagern hat den Vorteil, daß eine feinstufige Verschiebung der Linsen ausgeführt werden kann. Dieses erhöht die Exaktheit der gewünschten Fokuseinstellung und / oder des gewünschten Zoom-Faktors.

Um die räumliche Abbildung des Untersuchungsobjektes für eine weitergehende Bearbeitung zur Verfügung zu stellen, ist bei einer vorteilhaften Ausgestaltung der Erfindung vorgesehen, daß das Untersuchungsobjekt auf mehrere elektronische Speichermedien, die jeweils einem Strahlengang zugeordnet sind, räumlich abgebildet wird. Ebenso können natürlich die optischen. Strahlengänge direkt, über so genannte Tuben, den Augen des Betrachters zur Verfügung gestellt werden.

Eine bevorzugte Weiterbildung der Erfindung sieht vor, daß in den Strahlengängen ein jeweiliger Zoom-Faktor eingestellt wird, indem die erste Linsenanordnung, die zweite Linsenanordnung und / oder die Sammellinsenanordnung mit Hilfe der jeweiligen motorgetriebenen Verstelleinrichtung verlagert werden, wobei sich die jeweils einstellbaren Zoom-Faktoren der Strahlengänge voneinander unterscheiden. Auf diese Weise ist es ermöglicht, eine vergrößerte Anzeige eines der Kanäle auf einem Monitor oder einer Fotokamera zu erzeugen, wohingegen der andere Kanal eine geringere Vergrößerung aufweist, beispielsweise für die visuelle Betrachtung des Operateurs, wenn das optische System als ein Stereomikroskop ausgeführt ist.

Die Ausgestaltungen der Erfindung in den abhängigen Unteransprüchen zu dem optischen System mit einem Mikroskop weisen die in Verbindung mit den zugehörigen Merkmalen in den abhängigen Verfahrensansprüchen erläuterten Vorteile entsprechend auf.

Das optische System umfaßt vorzugsweise ein Stereomikroskop.

Als besonders vorteilhaft für eine exakte und fein abgestufte Verlagerung von Linsen in dem Mikroskop haben sich motorgetriebene Verlagerungseinrichtungen unter Verwendung eines bewegten magnetischen Wandelfeldes erwiesen, wie es beispielsweise in dem Dokument DE 103 23 629 A1 beschrieben ist.

### Beschreibung bevorzugter Ausführungsbeispiele der Erfindung

Die Erfindung wird im folgenden anhand von Ausführungsbeispielen unter Bezugnahme auf eine Zeichnung näher erläutert. Hierbei zeigen:
- Fig. 1: eine schematische Darstellung eines optischen Systems mit einem Stereomikroskop in Draufsicht; und
- Fig. 2: eine perspektivische Darstellung von optischen Linsen des Stereomikroskops in dem optischen System nach Fig. 1.

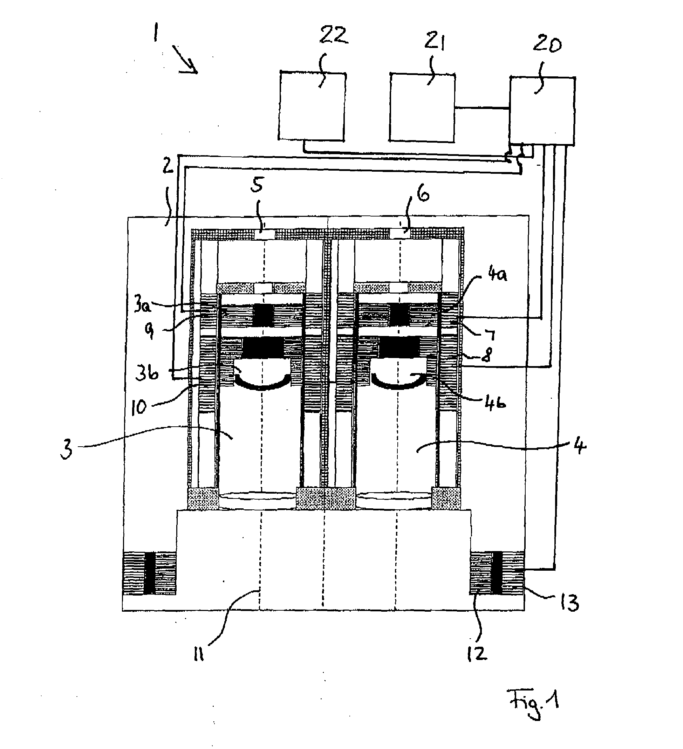

Fig. 1 zeigt eine schematische Darstellung eines optischen Systems 1 mit einem Stereomikroskop 2. In dem Stereomikroskop 2 sind zwei Strahlengänge 3, 4 gebildet. Mit Hilfe des Stcreomikroskops 2 wird ein Untersuchungsobjekt (nicht dargestellt) unter Nutzung der beiden Strahlengänge 3, 4 auf Videochips 5, 6 räumlich abgebildet. Die Videochips 5, 6 dienen als elektronische Speicher für die räumliche Abbildung, so daß anschließend eine Bildbearbeitung ausgeführt werden kann.

Entlang der optischen Achse in den beiden Strahlengängen 3, 4 sind jeweils eine erste und eine zweite Linsenanordnung 3a, 3b; 4a, 4b angeordnet. Die erste und die zweite Linsenanordnung 3a, 3b; 4a, 4h umfassen jeweils eine oder mehrere optische Linsen. Die optischen Linsen sind in einer jeweiligen Aufnahme gehalten, die ihrerseits mittels motorgetriebener Verstellglieder 7, 8, 9, 10 entlang der jeweiligen optischen Achse verlagerbar sind.

Den beiden Strahlengängen 3, 4 ist eine Sammellinsenanordnung 11 zugeordnet, die ebenfalls in einer Aufnahme 12 gehalten wird. In der Aufnahme 12 ist die Sammellinsenanordnung 11 mit Hilfe eines weiteren Verstellgliedes 13 verschiebbar.

Die motorgetriebenen. Verstellglieder 7, 8, 9, 10, 13 sind an eine Steuereinrichtung 20 gekoppelt, so daß von der Steuereinrichtung 20 erzeugte Steuersignale an die motorgetriebenen Verstellglieder 7, 8, 9,10, 13 übertragen werden können, um eine Fokuseinstellung und / oder eine Einstellung eines Zoom-Faktors des Stereomikroskops 2 vorzunehmen. Die Steuereinrichtung 20 ihrerseits ist mit einer Eingabeeinrichtung 21 verbunden, mit der Benutzereingaben erfaßt werden. Bei der Eingabeeinrichtung 21 handelt es sich beispielsweise um ein Tastfeld oder eine Tastatur, wie dieses im Rahmen von Bedienfeldern von Geräten bekannt ist.

Die motorgetriebenen Verstellglieder 7, 8, 9, 10, 13 sind drehfeldgetriebene Verstelleinrichtungen.

Die Baulänge des Stereomikroskops 2 ist dadurch reduziert, daß zur feinen Fokuseinstellung nicht nur die Sammellinsenanordnung 11 mittels des motorgetriebenen Verstellgliedes 13 bewegt wird, sondern auch die motorgetriebene Verstelleinrichtung 3a, 4a. Ein Zoom-Faktor des Stereomikroskops 2 wird mittels Verschieben der zweiten Linsenanordnung 3b, 4b erreicht. Die zweiten Linsenanordnungen 3b, 4b sind unabhängig voneinander einstellbar, so daß zu einem bestimmten Zeitpunkt in den beiden. Strahlengängen 3, 4 voneinander abweichende Zoom-Faktoren eingestellt werden können.

Wenn mit Hilfe der Eingabeeinrichtung 21 eine Benutzereingabe zum Einstellen der Fokuseinstellung und / oder des Zoom-Faktors des Stereomikroskops 2 erfaßt wird, werden anschließend in der Steuereinrichtung 20 Steuersignale entsprechend Vorabeinstellungsdaten erzeugt, die in einer mit der Steuereinrichtung 20 verbundenen Speichereinrichtung 22 elektronisch gespeichert sind. Es handelt sich hierbei um elektronische Informationen betreffend die Positionierung der ersten Linsenanordnung 3a, 4a und der zweiten Linsenanordnung 3b, 4b in einem oder beiden Strahlengängen 3, 4 und / oder der Sammellinsenanordnung 11 für bestimmte Fokuseinstellungen und / oder Zoom-Faktoren.

Wenn sich nach dem Erfassen der Benutzereingabe ergibt, daß zu der gewünschten Fokuseinstellung und / oder dem gewünschten Zoom-Faktor keine exakt passenden Vorcinstellungsdaten gespeichert sind, ermittelt die Steuereinrichtung 20 Voreinstellungsdaten für benachbarte Fokuseinstellungen / Zoom-Faktoren und interpoliert hieraus linear die Informationen zum Erzeugen der entsprechenden Steuersignale.

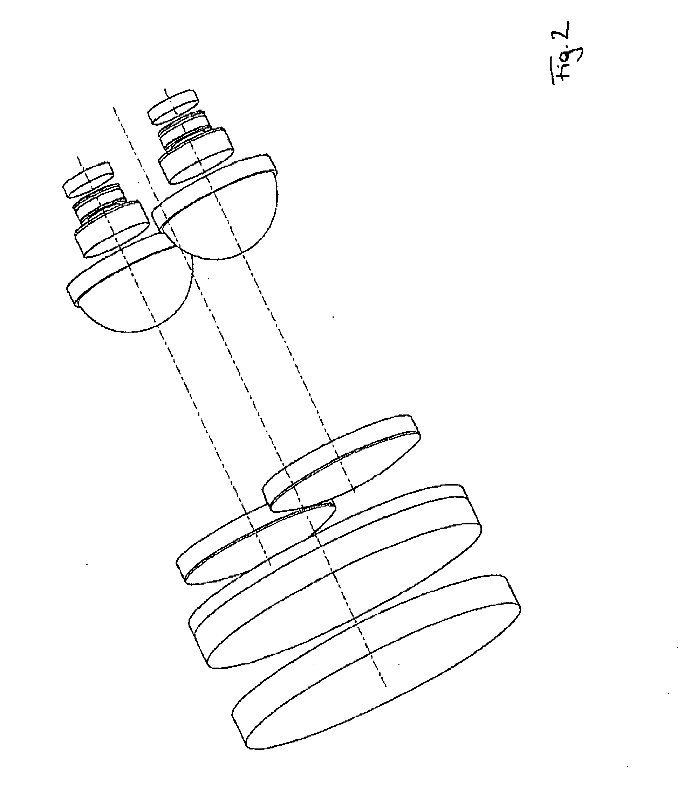

Fig. 2 zeigt eine perspektivische Darstellung von optischen Linsen der ersten und der zweiten Linsenanordnung 3a, 4a; 3b, 4b des Stereomikroskops 20 in dem optischen System nach Fig. 1.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Verfahren zum Betreiben eines optischen Systems (1), bei dem mittels eines von dem optischen System (1) umfaßten Mikroskops (2) ein Untersuchungsobjekt in eine Bildebene räumlich abgebildet wird, wobei das Mikroskop (2) mindestens ein Linsensystem, welches einem jeweiligen Strahlengang (3, 4) zugeordnet ist, und eine dem Strahlengang oder Strahlengängen (3, 4) zugeordnete Sammellinsenanordnung (11) aufweist, wobei das mindestens eine Linsensystem eine erste Linsenanordnung (3a; 4a) und eine zwischen der ersten Linsenanordnung (3a; 4a) und der Sammellinsenanordnung (11) angeordnete, zweite Linsenanordnung (3b; 4b) umfaßt, und wobei das Verfahren die folgenden Schritte umfaßt:
- Erfassen einer Benutzereingabe zum. Einstellen einer Fokuseinstellun.g und / oder eines Zoom-Faktors des Mikroskops (2) mit Hilfe einer Eingabeeinrichtung (21);
- Erzeugen von der erfaßten Benutzereingabe zugeordneten Steuersignalen zum Einstellen der Fokuseinstellung und / oder des Zoom-Faktors in einer an die Eingabeeinrichtung (21) gekoppelten Steuereinrichtung (20), wobei die Steuersignale Vorabeinstellungsdaten entsprechend gebildet werden, die in einer mit der Steuereinrichtung (20) verbundenen Speichereinnchtung (22) vorab gespeichert sind;
- Übertragen der Steuersignale an eine oder mehrere jeweiliger motorgetriebener Verstelleinrichtungen (7-10, 13) zum Verlagern der ersten Linsenanordnung (3a; 4a), der zweiten Linsenanordnung (3b; 4b) und / oder der Sammellinsenanordnung (11); und
- Einstellen der Fokuseinstellung und / oder des Zoom-Faktors des Mikroskops (2) entsprechend den Steuersignalen, indem die erste Linsenanordnung (3a; 4a), die zweite Linsenanordnung (3b; 4b) und / oder die Sammellinsenanordnung (11) mit Hilfe der jeweiligen motorgetriebenen Verstelleinochtung (7-10, 13) verlagert werden.

2. Verfahren nach Anspruch 1, **dadurch gekennzeichnet, daß** beim Einstellen der Fokuseinstellung eine grobe Fokuseinstellung ausgeführt wird, indem von den Steuersignalen umfaßte Grobsteuersignale in der Steuereinrichtung (20) erzeugt und von der Steuereinrichtung (20) an die motorgetriebene Verstelleinrichtung (13) der Sammellinsenanordnung (11) übertragen werden und die Sammellinsenanordnung (11) mit Hilfe der motorgetriebenen Verstelleinrichtung (13) der Sammellinsenanordnung (11) den Grobsteuersignalen entsprechend verlagert wird.

3. Verfahren nach Anspruch 1 oder 2, **dadurch gekennzeichnet, daß** beim Einstellen der Fokuseinstellung eine feine Fokuseinstellung ausgeführt wird, indem von den Steuersignalen umfaßte Feinsteuersignale in der Steuereinrichtung (20) erzeugt und von der Steuereinrichtung (20) an die jeweilige motorgetriebene Verstelleinrichtung (7; 9) der ersten Linsenanordnung (3a; 4a) in einem oder allen Strahlengängen (3, 4) übertragen werden und die erste Linsenanordnung (3a; 4a) in einem, oder allen Strahlengängen (3, 4) mit Hilfe der jeweiligen motorgetriebenen Verstelleinrichtung (7; 9) den Feinsteuersignalen entsprechend verlagert wird.

4. Verfahren nach einem der vorangehenden Ansprüche, **dadurch gekennzeichnet, daß** das Einstellen des Zoom-Faktors ausgeführt wird, indem von den Steuersignalen umfaßte Zoomsteuersignale in der Steuereinrichtung (20) erzeugt und von der Steuereinrichtung (20) an die jeweilige motorgetriebene Verstelleinrichtung (8; 10) der zweiten Linsenanordnung (3b; 4b) in einem oder allen Strahlengängen (3, 4) übertragen werden und die zweite Linsenanordnung (3b; 4b) in einem oder allen Strahlengängen (3, 4) mit Hilfe der jeweiligen motorgetriebenen Verstelleinrichtung (8; 10) den Zoomsteuersignalen entsprechend verlagert wird.

5. Verfahren nach einem der vorangehenden Ansprüche, **dadurch gekennzeichnet, daß** beim Erzeugen der Steuersignale zum Einstellen der Fokuseinstellung und / oder des Zoom-Faktors in der Steuereinrichtung (20) eine Interpolation zwischen ausgewählten Vorabeinstellungsdaten der gespeicherten Vorabeinstellungsdaten ausgeführt wird.

6. Verfahren nach einem der vorangehenden Ansprüche, **dadurch gekennzeichnet, daß** die Sammellinsenanordnung (11), die erste Linsenanordnung (3a; 4a) und / oder die zweite Linsenanordnung (3b; 4b) beim Verlagern entsprechend der Steuersignale mit Hilfe der jeweiligen motorgetriebenen Verstelleinnchtung (7-10, 13) linear verschoben werden.

7. Verfahren nach einem der vorangehenden Ansprüche, **dadurch gekennzeichnet, daß** mit den Steuersignalen in der jeweiligen motorgetriebenen Verstelleinrichtung (7-10, 13) ein Linearmotor beaufschlagt wird, welcher die den Steuersignalen entsprechende Verlagerung der Sammellinsenanordnung (11), der ersten Linsenanordnung (3 a; 4a) und / oder der zweiten Linsenanordnung (3b; 4b) bewirkt.

8. Verfahren nach einem der vorangehenden Ansprüche, **dadurch gekennzeichnet, daß** das Untersuchungsobjekt auf mehrere elektronische Speichermedien (5, 6), die jeweils einem Strahlengang (3; 4) zugeordnet sind, räumlich abgebildet wird, oder aber auch über Tuben direkt dem Betrachter zur Verfügung gestellt wird.

9. Verfahren nach einem der vorangehenden Ansprüche, **dadurch gekennzeichnet, daß** in den Strahlengängen (3, 4) ein jeweiliger Zoom-Faktor eingestellt wird, indem die erste Linsenanordnung (3a; 4a), die zweite Linsenanordnung (3b; 4b) und / oder die Sammellinsenanordnung (11) mit Hilfe der jeweiligen motorgetriebenen Verstelleinrichtung (7-10, 13) verlagert werden, wobei sich die jeweils einstellbaren Zoom-Faktoren der Strahlengänge (3, 4) voneinander unterscheiden.

10. Optisches Systems (1) mit einem Mikroskop (2) zum Abbilden eines Untersuchungsobjektes, wobei das Mikroskop (2) mindestens ein Linsensystem, welches einem jeweiligen Strahlengang (3, 4) zugeordnet ist, und eine dem jeweiligen Strahlengang oder Strahlengängen (3, 4) zugeordnete Sammellinsenanordnung (11) aufweist, und wobei das mindestens eine Linsensystem eine erste Linsenanordnung (3a; 4a) und eine zwischen der ersten Linsenanordnung (3a; 4a) und der Sammellinsenanordnung (11) angeordnete, zweite Linsenanordnung (3b; 4b) umfaßt, **gekennzeichnet durch**:
- eine Eingabeeinrichtung (21) zum Erfassen einer Benutzereingabe für eine einzustellende Fokuseinstellung und / oder einen einzustellenden Zoom-Faktor des Mikroskops (2);
- eine Steuereinrichtung (20), die an die Eingabeeinrichtung (21) gekoppelt ist und mit welcher der erfaßten Benutzereingabe zugeordnete Steuersignale zum Einstellen der Fokuseinstellung und / oder des Zoom-Faktors erzeugbar sind, wobei die Steuersignale Vorabeinstellungsdaten entsprechend gebildet werden, die in einer mit der Steuereinrichtung (209 verbundenen Speichereinrichtung (22) vorab gespeichert sind;
- eine jeweilige motorgetriebene Verstelleinrichtung (7-10, 13) der ersten Lmsenanordnung (3a; 4a), der zweiten. Linsenanordnung (3b; 4b) und / oder der Sammellmsenanordnung (11), mit denen die erste Linsenanordnung (3a; 4a), die zweite Linsenanordnung (3b; 4b) und oder die Sammellinsenanordnung (11) den Steuersignalen entsprechend einstellbar sind; und
- Übertragungsrvittel zum Übertragen der Steuersignale an eine oder mehrere der jeweiligen motorgetriebenen Verstelleinrichtung (7-10, 13) der ersten. Linsenanordnung (3a; 4a), der zweiten Linsenanordnung (3b; 4b) und / oder der Sammellmsenanordnung (11).

11. Optisches System nach Anspruch 10, **dadurch gekennzeichnet, daß** die motorgetriebene Verstelleinrichtung (13) der Sammellinsenanordnung (11) einer groben Fokuseinstellung zugeordnet ist, so daß mit der motorgetriebenen Verstelleinrichtung (13) der Sammellinsenanordnung (11) von der Steuereinrichtung (20) empfangene und von den Steuersignalen umfaßte Grobsteuersignale verarbeitbar sind.

12. Optisches System nach Anspruch 10 oder 11, **dadurch gekennzeichnet, daß** die motorgetriebene Verstelleinrichtung (7; 9) der ersten Linsenanordnung (3a; 4a) in einem oder allen Strahlengängen (3, 4) einer feinen Fokuseinstellung zugeordnet ist, so daß mit der motorgetriebenen Verstelleinrichtung (7; 9) der ersten Linsenanordnung (3a; 4a) in einem oder allen Strahlengängen. (3, 4) von der Steuereinrichtung (20) empfangene und von den Steuersignalen umfaßte Feinsteuersignale zum Einstellen der feinen Fokuseinstellung verarbeitbar sind.

13. Optisches System nach einem der Ansprüche 10 bis 12, **dadurch gekennzeichnet, daß** die motorgetriebene Verstelleinrichtung (8; 10) der zweiten Linsenanordnung (3b; 4b) in einem oder allen Strahlengängen (3, 4) dem Zoom-Faktor zugeordnet ist, so daß mit der motorgetriebenen Verstelleinrichtung (8; 10) der zweiten Linsenanordnung (3b; 4b) in einem oder allen Strahlengängen (3, 4) von der Steuereinrichtung (20) empfangene und von den Steuersignalen umfaßte Zoomsteuersignale zum Einstellen des Zoom-Faktors verarbeitbar sind.

14. Optisches System nach einem der Ansprüche 10 bis 13, **dadurch gekennzeichnet, daß** die Steuereinrichtung (20) Interpolationsmittel umfaßt, mit denen beim Erzeugen der Steuersignale zum Einstellen der Fokuseinstellung und / oder des Zoom-Faktors in der Steuereinrichtung (20) eine Interpolation zwischen ausgewählten Vorabeinstellungsdaten der gespeicherten Vorabeinstellungsdaten ausführbar ist.

15. Optisches System nach einem der Ansprüche 10 bis 14, **dadurch gekennzeichnet, daß** die jeweilige motorgetriebene Verstelleinrichtung (7-10, 13) der Sammellinsenanordnung (11), der ersten Linsenanordnung (3a; 4a) und / oder der zweiten Linsenanordnung (3b; 4b) einen Linearmotor umfaßt.

16. Optisches System nach einem der Ansprüche 10 bis 15, **dadurch gekennzeichnet, daß** der Sammellinsenanordnung (11), der ersten Linsenanordnung (3a; 4a) und / oder der zweiten Linsenanordnung (3b; 4b) in einer oder mehreren Hülsen angeordnet und mittels eines bewegten magnetischen Wandelfeldes in einer oder allen Hülsen jeweils verlagerbar sind.

17. Optisches System nach einem der Ansprüche 9 bis 15, **gekennzeichnet durch** mehrere elektronische Speichermedien (5, 6), die jeweils einem Strahlengang (3; 4) zugeordnet sind, zum Aufnehmen der räumlichen Abbildung des Untersuchungsobjektes.

18. Optisches System nach einem der Ansprüche 10 bis 17, **dadurch gekennzeichnet, daß** die jeweilige motorgetriebene Verstelleinrichtung (7-10, 13) der Sammellinsenanordnung (11), der ersten Linsenanordnung (3a; 4a) und / oder der zweiten Linsenanordnung (3b; 4b) drehfeldgetrieben sind.

19. Optisches System nach einem der Ansprüche 10 bis 18, **dadurch gekennzeichnet, daß** mittels der jeweiligen motorgetriebenen. Verstelleinrichtung (7-10,13) der Sammellinsenanordnung (11), der ersten Linsenanordnung (3a; 4a) und / oder der zweiten Linsenanordnung (3b; 4b) in den Strahlengängen (3, 4) ein jeweiliger Zoom-Faktor einstellbar ist, wobei sich die jeweils einstellbaren Zoom-Faktoren der Strahlengänge (3, 4) voneinander unterscheiden.
